(19) Europäisches Patentamt / European Patent Office / Office européen des brevets

(11) **EP 2 978 383 B1**

(12) **EUROPEAN PATENT SPECIFICATION**

(45) Date of publication and mention of the grant of the patent:
**15.11.2023 Bulletin 2023/46**

(21) Application number: **14714675.7**

(22) Date of filing: **28.03.2014**

(51) International Patent Classification (IPC):
$C03C\ 1/00^{(2006.01)}$  $C03C\ 3/062^{(2006.01)}$
$C03C\ 3/11^{(2006.01)}$  $C03C\ 3/112^{(2006.01)}$
$C03C\ 4/12^{(2006.01)}$  $C03C\ 10/00^{(2006.01)}$
$C03C\ 10/16^{(2006.01)}$  $C03C\ 4/00^{(2006.01)}$
$C03C\ 12/00^{(2006.01)}$  $A61K\ 8/25^{(2006.01)}$
$A61L\ 27/10^{(2006.01)}$  $A61L\ 27/12^{(2006.01)}$
$A61Q\ 11/00^{(2006.01)}$  $B24C\ 11/00^{(2006.01)}$
$A61C\ 3/025^{(2006.01)}$

(52) Cooperative Patent Classification (CPC):
**A61Q 11/00; A61K 8/25; A61L 27/10; A61L 27/12; B24C 11/00; C03C 1/002; C03C 3/062; C03C 3/11; C03C 3/112; C03C 4/0007; C03C 4/0014; C03C 4/0021; C03C 4/12; C03C 10/0063; C03C 10/16;** (Cont.)

(86) International application number:
**PCT/EP2014/056318**

(87) International publication number:
**WO 2014/154874 (02.10.2014 Gazette 2014/40)**

(54) **CHLORINE-CONTAINING SILICATE GLASSES AND GLASS CERAMICS**

CHLORHALTIGE SILICATGLÄSER UND GLASKERAMIKEN

VERRES DE SILICATE ET VITROCÉRAMIQUES CONTENANT DU CHLORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2013 GB 201305774**

(43) Date of publication of application:
**03.02.2016 Bulletin 2016/05**

(73) Proprietor: **Queen Mary and Westfield College University of London**
**London E1 4NS (GB)**

(72) Inventors:
• **HILL, Robert**
  **London E1 4NS (GB)**
• **KARPUKHINA, Natalia**
  **London E1 4NS (GB)**
• **CHEN, Xiaojing**
  **London E1 4NS (GB)**

(74) Representative: **Lock, Graham James et al**
**Fry Heath & Spence LLP**
**Unit A, Faraday Court**
**Faraday Road**
**Crawley, West Sussex RH10 9PU (GB)**

(56) References cited:
WO-A1-96/25203  WO-A1-98/43922
CN-A- 1 438 192  JP-A- H10 291 835
US-A- 5 804 520  US-A1- 2007 170 396

• SANDLAND T O ET AL: "Structure of Cl-containing silicate and aluminosilicate glasses: A <35>Cl MAS-NMR study", GEOCHIMICA ET COSMOCHIMICA ACTA, PERGAMON PRESS, NEW YORK, NY, US, vol. 68, no. 24, 15 December 2004 (2004-12-15), pages 5059-5069, XP004686527, ISSN: 0016-7037, DOI: 10.1016/J.GCA.2004.07.017

EP 2 978 383 B1

- KITHERI JOSEPH ET AL: "Studies on Novel Matrices for High Level Waste from Fast Reactor Fuel Reprocessing", ENERGY PROCEDIA, vol. 7, 7 June 2011 (2011-06-07), pages 518-524, XP028269382, ISSN: 1876-6102, DOI: 10.1016/J.EGYPRO.2011.06.071 [retrieved on 2011-08-22]
- METCALFE B L ET AL: "Candidate wasteforms for the immobilization of chloride-containing radioactive waste", JOURNAL OF NON-CRYSTALLINE SOLIDS, NORTH-HOLLAND PHYSICS PUBLISHING. AMSTERDAM, NL, vol. 348, 15 November 2004 (2004-11-15), pages 225-229, XP004645376, ISSN: 0022-3093, DOI: 10.1016/J.JNONCRYSOL.2004.08.173

(52) Cooperative Patent Classification (CPC): (Cont.)
**C03C 12/00;** A61C 3/025; A61L 2430/02

**Description**

The present invention relates to chlorine-containing silicate glasses and glass ceramics. Glasses are entirely amorphous (i.e non-crystalline), whereas glass ceramics have an amorphous phase and one or more crystalline phases. Glass ceramics are prepared by heating a glass until it has been partially crystallized.

The incorporation of chlorine in silicate glasses is problematic due to chlorine volatilization during melting, resulting in the loss of most of the chlorine. As a consequence, chlorine is rarely incorporated into silicate glasses, and there are currently no commercial applications of chlorine-containing silicate glasses. Although the same problem may occur with regards to the incorporation of fluorine, it is not as severe. Accordingly, fluorine-containing glasses and glass ceramics are fairly common.

Whilst chlorine is likely to behave like fluorine within the glass structure and confer similar properties on the glass, the chloride ion is substantially larger than the fluoride ion (0.167nm cf 0.119nm), and the applicant believes that this has important and significant consequences. Thus, chlorine-containing glasses are less likely to crystallise during synthesis compared to the equivalent fluoride glass. Furthermore, as the chloride ion is much larger then the oxygen ion (0.167nm cf 0.126nm), the introduction of chlorine would be expected to expand the glass network compared to the equivalent fluoride glass resulting in a more open structure that might be expected to result in lower moduli, lower hardness values, lower glass transition temperatures, lower melting temperatures, and also result in more degradable glasses.

It would be attractive to be able to incorporate chlorine into silicate glasses for a wide range of commercial applications including bioactive glasses, glass ceramics, radioactive waste vitrification, and glass ceramics for up conversion applications.

A biologically active (or bioactive) material is one which, when implanted into living tissue, induces formation of an interfacial bond between the material and the surrounding tissue. Bioactive glasses are a group of surface-reactive glasses which exhibit bioactivity. The bioactivity of these glasses is the result of complex reactions which take place on the surface of the glass under physiological conditions, and which result in the formation of hydroxycarbonated apatite (HCA) on the surface of the glass.

Because of the ability of bioactive glasses to bond with living tissue, and in particular bone and tooth, they are used in a number of medical applications, including dental applications such as toothpaste.

For many applications, and in particular for toothpastes, it is preferable that the glass should release fluoride and form fluorapatite (FAP), instead of HCA. This is because FAP is more resistant to acid dissolution in oral fluids than HCA and aids in the prevention of dental caries. Moreover, fluoride ions are known to aid apatite formation and stimulate the cell division of osteoblasts, the bone forming cells. For these reasons, fluoride has been incorporated into bioactive glasses.

Although fluoride has been incorporated into bioactive glasses, there are some disadvantages. For example, fluoride is classified as a drug by the Federal Drug Administration in the USA, making it more difficult to obtain approval for glass-based medical and dental applications containing fluorine. Thus, fluoride-containing toothpastes are classified as a drug in the US, whilst in Europe they are classified as a cosmetic. In addition, fluoride ions are not naturally present in the body in significant concentrations.

Chloride does not have the disadvantage of fluoride in bioactive glasses of forming fluorite ($CaF_2$) or the equivalent chloride when its concentration in the glass is above 5 mol% and it does not cause fluorosis. Thus, chloride is not classified as a drug by the Federal Drug Administration in the USA. Moreover, chloride ions are found naturally in the body in significant concentrations. For these reasons, it would be desirable to be able to make chlorine-containing glasses for use in glass-based medical and dental applications in place of fluorine-containing glasses.

Although a fluorine-containing glass forms fluorapatite under physiological conditions, the corresponding chlorine-containing glass will not form chlorapatite under such conditions.

This is because the chloride ion is larger than the hydroxyl ion, with the result that the hydroxyl ion goes into the crystal lattice. The applicant has evidence that the substance thus formed is not a hydroxyapatite, but an "apatite like" phase most probably octacalcium phosphate with a chemical formula close to $Ca_3(PO_4)_6H_2.5H_2O$. By comparison, hydroxyapatite has the formula $Ca_{10}(PO_4)_6(OH)_2$. These two phases exhibit almost identical X-ray diffraction patterns and infra red spectra and are hard to distinguish apart. The applicant believes that this means that octacalcium phosphate is often misidentified as hydroxyapatite and hydroxycarbonated apatite. Octacalcium phosphate unlike hydroxyapatite has a water layer in its crystal structure, two acidic phosphate residues and lacks the hydroxyl ion of hydroxyapatite. So whilst it has structural features of hydroxyapatite, it is actually quite different. Octacalcium phosphate can only be distinguished readily and reliably from hydroxyapatite by means of $^{31}P$ solid state NMR spectroscopy which is not widely available.

An example of a glass-based medical/dental application where chlorine-containing glasses could replace fluorine-containing glasses is toothpaste. For example, fluorine-containing glasses may be used in toothpaste as re-mineralising additives. The glass dissolves in the mouth releasing calcium phosphate and fluoride ions and forms apatite on the tooth surface, so re-mineralising the tooth surface and incipient caries lesions. This will occlude exposed dentinal tubules and treat dentine hypersensitivity.

A further example of such an application is air abrasion. In air abrasion, glass particles are injected into a high pressure air stream and used to cut or polish teeth. Air abrasion offers many benefits over the use of conventional dental drills for cutting cavities, cleaning and polishing and removal of resin from teeth after debonding orthodontic brackets in that it doesn't result in any subsurface damage to the tooth structure.

Glass ceramics which form fluorapatite are also attractive for medical and dental applications. Since hydroxyl ions can not be incorporated into glasses, hydroxyapatite glass ceramics can not be formed readily. Fluorides are incorporated into the glass and the fluorine analogue of hydroxyapatite, fluorapatite, can be crystallised from glasses upon heat treatment. Fluorapatite is chemically much more stable than hydroxyapatite and much more stable than hydroxycarbonated apatite, consequently fluorapatite glass ceramics can not be resorbed easily and are suitable for permanent non-resorbable implant materials. Since the chloride ion is larger than the hydroxyl ion and much larger still than the fluoride ion, chloroapatite should be more resorbable than fluorapatite or even hydroxyapatite. Accordingly, chlorapatite glass ceramics could be used as implant materials in place of fluorapatite glass ceramics.

Apatites are attractive for radioactive waste vitrification since the apatite crystal structure is very accommodating of other ions, and apatites exhibit extensive solid solution phase behavior. However, apatites are not particulary stable to dissolution and the powders produced are difficult to sinter to form a monolith. Because of the problems associated with chlorine volatilization from silicate melts, the nuclear waste industry has extensively investigated converting chlorine-containing radioactive wastes into mixed fluorochloroapatites by solid state reaction, as direct vitrification is not feasible. This has led Vance et al. (J. Nuclear Materials 420 (2012) 396-404.) to propose that chloroapatite glass ceramics would be attractive waste forms. However such glass ceramics do not yet exist and there still remains the problem of chlorine volatilization during melting to be overcome.

WO 96/25203 A1 discloses chloride comprising glasses useful for hazardous waste vitrification.

Due to their low phonon energies and their ability to incorporate other ions such as manganese, antimony or rare earths and form extensive solid solutions, fluorapatites are attractive as up conversion processors. Mixed fluor/chlorocalcium/strontium apatites $(Ca/Sr_5(PO_4)_3F/Cl)$ are widely used already as commercial lamp phosphors in fluorescent tubes. The tube produces short wavelength ultraviolet light, which is absorbed by the apatite phosphor, which re-emits the light into the visible part of the spectrum. The absorption-emission spectrum depends on the dopant ion, but because apatites exhibit complete solid solution phase behavior with regard to chlorine/fluorine and calcium/strontium, the absorption-emission spectrum can be tuned by varying the substitution in the lattice.

Optically transparent glass ceramics based on mixed fluoro/chloroapatites where the crystal phase is kept to dimensions much less than the wavelength of light are particularly attractive for up conversion applications including laser fibre amplifiers and coatings on silicon based solar cells to increase solar efficiency. Here a coating on the silicon absorbs short wavelength light not available to the solar cell and re-emits it into the spectral range usable by the solar cell.

It is interesting to note that this process of up conversion or fluorescence also occurs very efficiently in natural teeth, which are also based on solid substituted apatites. This phenomena makes teeth appear whiter in natural light containing short wavelengths. Improving this process in order to satisfy the demand for "whiter" teeth would be much preferable to current tooth whitening procedures involving bleaching and peroxides, which destroy the protein component of teeth.

From the above, it can be seen that there are many possible applications of chlorine-containing silicate glasses and glass ceramics. In order for these applications to be put into practice, however, it is necessary to overcome the problem of chlorine volatilization during melting, resulting in the loss of most of the chlorine.

It is an object of the invention to address the problem of chlorine volatilization in chlorine-containing silicate glasses.

Accordingly, the invention provides a chlorine-containing silicate glass as from claim 1.

The at least 10 mole percent of MgO, SrO, BaO and CaO may consist entirely of any one of MgO, SrO, BaO or CaO, or may consist of a combination of any of MgO, SrO, BaO and/or CaO.

Silicate glasses may contain "bridging oxygens" and "non-bridging oxygens". A "bridging oxygen" is an oxygen that is shared by two $SiO_4$ tetrahedra. A "non-bridging oxygen" is any oxygen that is not shared by two $SiO_4$ tetrahedra.

The applicant has surprisingly found that, for silicate glasses which contain little or no $Al_2O_3$, chlorine volatilization is suppressed in glasses which have a relatively high non-bridging oxygen content. This effect is lost, however, if the non-bridging oxygen content is too high. Accordingly, where the glass comprises less than 1 mole percent $Al_2O_3$, the ratio of non-bridging oxygens to bridging oxygens is preferably between 0.7 and 1.5.

Network connectivity (NC) is defined as the average number of non-bridging oxygens per silicon. For silicate glasses which contain little or no $Al_2O_3$, it has been found that chlorine volatilization is markedly suppressed where the glass has a network connectivity of two, that is, where there are approximately two non-bridging oxygens per silicon corresponding to a $Q^2$ silicate structure. However, attempts to make chlorine-containing glasses with a network connectivity of three or more resulted in marked chlorine loss, and little or no retention of chlorine in the glass once it has been melted.

Accordingly, the silicate glasses contain less than 1 mole percent $Al_2O_3$, the glass has a network connectivity is in the range 2 to 3.

For silicate glasses containing less than 1 mole percent $Al_2O_3$, network connectivity may be calculated as follows:

$$NC = ((4*[SiO_2]) - (2*([R_2O]+[RO])-(6*[P_2O_5]))/[SiO_2]$$

In the above equation, the concentrations defined in square brackets are expressed in mole fractions in the glass and RO represents an alkaline earth metal oxide and $R_2O$ an alkali metal oxide.

The glass comprises at least 1.5 mole percent metal chloride, more preferably at least 2 mole percent metal chloride.

The glass may comprise up to 50 mole percent metal chloride.

The glass may comprise up to 60 mole percent of MgO, SrO, BaO, and CaO combined.

Preferably, the glass comprises either CaO, SrO or BaO and a source of phosphate. Compositions containing either CaO, SrO or BaO and a source of phosphate have been found to crystallise to chloroapatite on heat treatment, which is attractive for the wide range of applications discussed above.

The glass may comprise at least 20 mole percent combined of CaO and SrO. Barium is toxic, and so CaO and SrO are preferred over BaO for medical and dental applications.

The glass may comprise a fluoride. Compositions containing both chlorine and fluorine crystallise to fluorapatite if there is more fluorine than the stoichiometry of fluorapatite. Compositions containing both chlorine and fluorine where the fluorine is present in amounts below the stoichiometry of fluorapatite crystallise to a mixed fluorochloroapatite. The applicant believes that the fluoride ion goes into the apatite lattice more readily than the larger chloride ion. This is probably a result of the smaller size of the fluoride ion and its ability to occupy the space in the CaII triangle in the apatite lattice.

The glass may comprise at least 20 mole percent $SiO_2$.

The glass may comprise up to 60 mole percent $SiO_2$, preferably up to 50 mole percent $SiO_2$.

The glass may comprise at least 2 mole percent $P_2O_5$, preferably at least 4 mole percent $P_2O_5$.

The glass may comprise up to 20 mole percent $P_2O_5$, preferably up to 12 mole percent $P_2O_5$, more preferably up to 10 mole percent $P_2O_5$ and most preferably up to 8 mole percent $P_2O_5$.

For silicate glasses which do contain $Al_2O_3$, the applicant has surprisingly found that chlorine volatilization is suppressed at a lower level of non-bridging oxygens. Whilst not wishing to be limited by theory, the applicant believes that this is because there is a different mechanism for chlorine retention in alumina-containing glasses. The applicant has found that the solid state NMR of fluorine-containing glasses indicates that there are two major fluorine species, an $Al-F-Ca_{(n)}$ species that represents a fluorine bound to Al with an associated $Ca^{2+}$ ion to maintain the charge balance, and an $F-Ca_{(n)}$ species. Accordingly, the applicant believes that the formation of Al-F bonds minimizes the chances of formation of Si-F bonds, and so minimizes the chances of formation of volatile $SiF_4$. The same applies to chlorine-containing glasses, so that, where alumina present, there is less loss of chlorine through formation of volatile $SiCl_4$.

Not according with the invention the glass comprises at least 1 mole percent $Al_2O_3$, preferably at least 2 mole percent $Al_2O_3$, the glass preferably comprises 8 to 60 mole percent of alkaline earth oxide and alkaline earth chloride or fluoride combined, the glass comprising at least 2 mole percent metal chloride and metal fluoride combined.

Preferably, the glass comprises either CaO, SrO or BaO and a source of phosphate. As discussed above, compositions containing either CaO, SrO or BaO and a source of phosphate have been found to crystallise to chloroapatite on heat treatment.

The glass may comprise 2 to 12 mole percent MgO, $MgCl_2$ or $MgF_2$.

The apatite-forming glasses of the invention may be used for various dental applications, including promoting re-mineralisation, preventing or treating caries, periodontal disease or dentine hyper sensitivity or tooth whitening. They may also be used as a bone substitute, for example, in restoring or repairing tooth or bone structure.

Glass ceramics are prepared by heating a glass until it has been partially crystallized. Accordingly, the invention further provides a glass ceramic prepared by subjecting a glass according to the invention to heat treatment.

The glass may be heated to a temperature between 300 and 900°C. Where the glass comprises either CaO, SrO or BaO and a source of phosphate, the glass may be heated until it crystallises to an apatite phase. Where the glass does not contain any fluorine, the apatite will be a chloroapatite. Where the glass does contain fluorine, but in amounts below the stoichiometry of fluorapatite, then the apatite will be a mixed fluoro/chloroapatite. The chloroapatite or mixed fluorochloroapatite may have dimensions less than 500nm, preferably less than 100nm.

The apatite-containing glass ceramics of the invention may be used for various dental applications, including promoting re-mineralisation, preventing or treating caries, periodontal disease or dentine hyper sensitivity or tooth whitening. They may also be used as a bone substitute, for example, in restoring or repairing tooth or bone structure.

In addition, they may be used in radioactive waste vitrification, particularly for chlorine-containing wastes.

Furthermore, they may be used in up conversion applications. For example, they may be used as a fibre laser amplifier. For this application, they should be doped with one or more rare earth elements, wherein the rare earths may be selected from Y, La, CePr, Pm, Sm, Eu, Gd,Tb, Dy, Ho ,Er, Tm ,Yb and Lu, or with transition elements including Mn that exhibit fluorescence. They may also be used as lamp phosphors. For this purpose, they should also be doped with a rare earth element or transition element.

The present invention further provides a toothpaste comprising an apatite-forming glass according to the invention or an apatite-containing glass ceramic according to the invention.

A number of specific embodiments of the invention will now be described by way of example only with reference to the accompanying drawings of which:

Figure 1 shows a typical DSC trace for a glass frit of QMXJC16, a $CaX_2$ series glass;

Figure 2 shows the glass transition temperatures for three series of glasses, a $CaCl_2$ series, a $CaF_2$ series and a $CaF_2$ .$CaCl_2$ series;

Figure 3 shows a DSC trace for frit and <38 micron powder of W6; and

Figure 4 shows the FITR spectra of QMXJC16, a $CaX_2$ series glass.

Example 1

High purity quartz (i.e. >99.99%$SiO_2$) (46.35g), Calcium carbonate (112.53g), anhydrous calcium chloride (23.05g), and phosphorus pentoxide (18.07g) were weighed out and mixed thoroughly together. The mixture was then placed in a 300ml platinum/rhodium crucible and the crucible heated to 1450°C for one hour. The crucible was then removed from the furnace and the resulting melt quenched rapidly into demineralized water. The resulting glass was removed immediately, washed with ethanol and dried at 120°C for one hour.

The resulting granular glass was ground in a percussion mill and sieved through a 38 micron sieve.

Example 2

The preparation method used in Example 1 may be used to prepare other glasses using different components. For example, the calcium carbonate may be partially replaced by other alkali metals or alkali earth carbonates, and the phosphate may be incorporated in the form of a variety of phosphates. In addition, the calcium chloride may be replaced by other alkali earths or alkali metal chlorides.

Table 1 shows a range of glass compositions prepared using the preparation method of Example 1. Glasses W1, QMRW1-4 and QMUP1-4 are not according with the invention.

Table1

| Glass | $SiO_2$ | $Al_2O_3$ | MgO | CaO | SrO | $Na_2O$ | $P_2O_5$ | $CaF_2$ | $CaCl_2$ |
|-------|---------|-----------|-----|-----|-----|---------|----------|---------|----------|
| W1 | 50 | 0 | 0 | 50 | 0 | 0 | 0 | 0 | 0 |
| W2 | 48.54 | 0 | 0 | 48.54 | 0 | 0 | 0 | 0 | 2.91 |
| W3 | 47.85 | 0 | 0 | 47.85 | 0 | 0 | 0 | 0 | 4.31 |
| W4 | 47.17 | 0 | 0 | 47.17 | 0 | 0 | 0 | 0 | 5.66 |
| W5 | 45.75 | 0 | 0 | 45.75 | 0 | 0 | 0 | 0 | 8.51 |
| W6 | 44.01 | 0 | 0 | 44.01 | 0 | 0 | 0 | 0 | 11.97 |
| W7 | 42.44 | 0 | 0 | 42.44 | 0 | 0 | 0 | 0 | 15.11 |
| W8 | 39.84 | 0 | 0 | 39.84 | 0 | 0 | 0 | 0 | 20.32 |
| QMXJ16 | 37 | 0 | 0 | 53.9 | 0 | 0 | 6.1 | 0 | 3 |
| QMXJ17 | 36.4 | 0 | 0 | 53 | 0 | 0 | 6 | 0 | 4.5 |
| OMXJ18 | 35.9 | 0 | 0 | 52.2 | 0 | 0 | 6 | 0 | 6 |
| QMXJ19 | 34.6 | 0 | 0 | 50.4 | 0 | 0 | 5.7 | 0 | 9.3 |

(continued)

| Glass | $SiO_2$ | $Al_2O_3$ | MgO | CaO | SrO | $Na_2O$ | $P_2O_5$ | $CaF_2$ | $CaCl_2$ |
|---|---|---|---|---|---|---|---|---|---|
| QMXJ20 | 32.9 | 0 | 0 | 48 | 0 | 0 | 5.5 | 0 | 13.60 |
| QMXJ21 | 31.4 | 0 | 0 | 45.7 | 0 | 0 | 5.2 | 0 | 17.8 |
| QMXJ22 | 28.4 | 0 | 0 | 41.4 | 0 | 0 | 4.7 | 0 | 25.5 |
| QMXJ23 | 37 | 0 | 0 | 53.9 | 0 | 0 | 6.1 | 1.5 | 1.5 |
| GCB1 | 37 | 0 | 0 | 40 | 0 | 13.9 | 6.1 | 0 | 3 |
| GCB2 | 37 | 0 | 0 | 30 | 10 | 13.9 | 6.1 | 1 | 2 |
| GCB3 | 37 | 0 | 0 | 30 | 10 | 13.9 | 6.1 | 0 | 3 |
| QMX24 | 36.4 | 0 | 0 | 53 | 0 | 0 | 6 | 2.25 | 2.25 |
| OMXJ25 | 35.9 | 0 | 0 | 52.2 | 0 | 0 | 6 | 3 | 3 |
| QMXJ26 | 34.6 | 0 | 0 | 50.4 | 0 | 0 | 5.7 | 4.65 | 4.65 |
| QMXJ27 | 32.9 | 0 | 0 | 48 | 0 | 0 | 5.5 | 6.8 | 6.8 |
| QMXJ28 | 31.4 | 0 | 0 | 45.7 | 0 | 0 | 5.2 | 8.9 | 8.9 |
| QMXJ29 | 28.4 | 0 | 0 | 41.4 | 0 | 0 | 4.7 | 12.25 | 12.25 |
| TP1 | 28.4 | 0 | 0 | 41.4 | 0 | 0 | 4.7 | 4 | 21 |
| TP2 | 28.4 | 0 | 0 | 21.4 | 0 | 20 | 4.7 | 4 | 21 |
| QMRW1 | 36 | 6 | 0 | 38 | 0 | 0 | 5 | 0 | 15 |
| QMRW2 | 35 | 6 | 8 | 30 | 0 | 0 | 5 | 0 | 16 |
| QMRW3 | 34 | 8 | 5 | 32 | 0 | 0 | 5 | 0 | 16 |
| OMRW4 | 35 | 8 | 5 | 32 | 0 | 0 | 5 | 3 | 12 |
| OMUP1 | 35 | 11 | 10 | 36 | 0 | 0 | 6 | 1 | 1 |
| QMUP2 | 35 | 11 | 10 | 36 | 0 | 0 | 6 | 1.5 | 0.5 |
| QMUP3 | 35 | 11 | 10 | 18 | 18 | 0 | 6 | 1 | 1 |
| QMUP4 | 40 | 6 | 10 | 18 | 18 | 0 | 6 | 1 | 1 |
| Glass | $SiO_2$ | $Al_2O_3$ | MgO | CaO | SrO | $Na_2O$ | $P_2O_5$ | $CaF_2$ | $CaCl_2$ |

Each of the glass composition powders produced was subjected to X-ray powder diffraction. All of the chlorine-containing glasses with no fluorine were amorphous.

Differential Scanning Calorimetry (DSC) was performed on both the <38 micron powder and the granular glass produced on quenching. Figure 1 shows a typical DSC trace. Marked on the trace is the glass transition temperature (Tg) and the peak crystallization temperature (Tc).

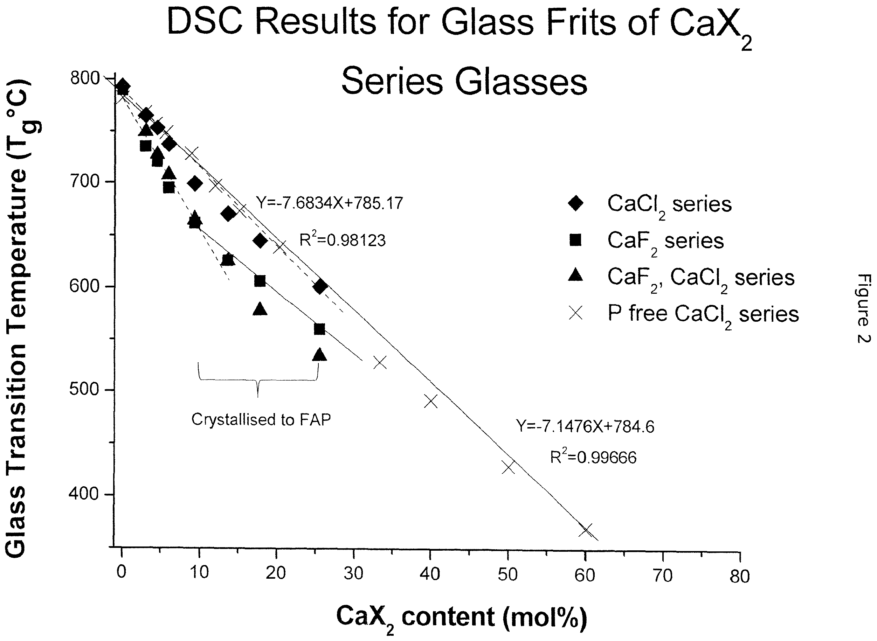

Figure 2 shows the glass transition temperatures for three series of glasses. The crosses show phosphate-free glasses based on Wollastonite $CaSiO_3$ where $CaCl_2$ has been added to the composition. The diamonds show the 6 mole% $P_2O_5$ glasses with $CaCl_2$. The triangles show mixed halide glasses containing fluorine and chlorine in equal amounts. The squares show the fluorine-containing glasses. Note the deviation from linearity for fluorine-containing glasses corresponds with undesirable crystallization of the glass to fluorapatite during quenching. With mixed fluoride/chlorine-containing glasses this is suppressed to higher $CaX_2$ contents and with chlorine only containing glasses this undesirable crystallization does not occur. The first crystallization peak corresponds to the formation of chloroapatite in the chlorine containing glasses and to fluorapatite in the fluorine-containing glasses.

X-ray diffraction was performed after heat treating the glasses to the temperature corresponding to the peak crystallization temperatures observed from the DSC traces in the temperature range 400 to 950°C. In all cases with 6 mole% $P_2O_5$ in the glass, the phase detected was an apatite. In the absence of fluorine and the presence of chlorine, chloroapatite formed on heat treatment.

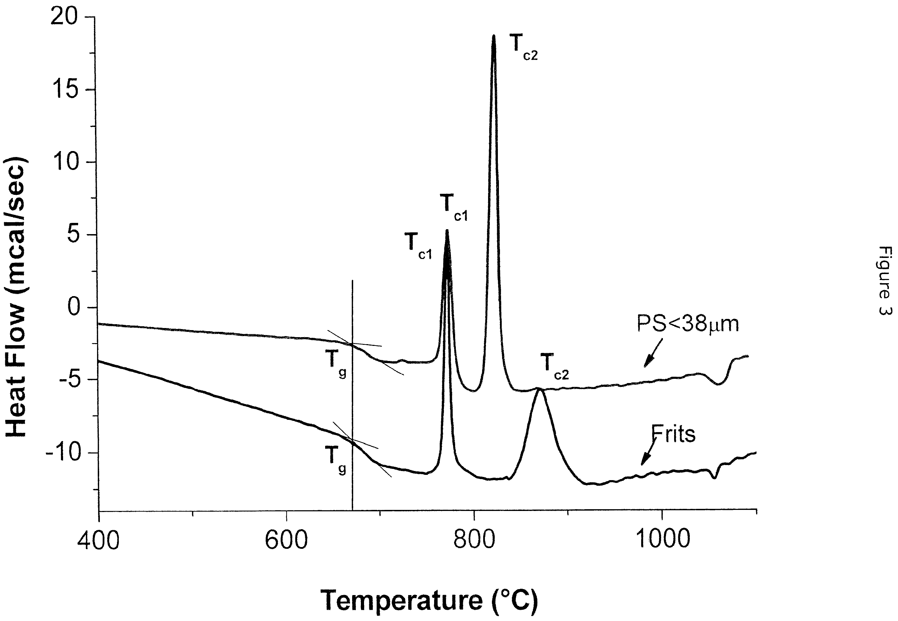

One of the key aspects of a glass ceramic is the ability to crystallize from the bulk as opposed to from the surface. Surface crystallization induces residual surface stresses that often result in cracking in terms of processing, whereas bulk crystallisation, otherwise known as bulk crystal nucleation, is a prerequisite for obtaining small well dispersed crystals and is also of critical importance in forming nanocrystals for optically transparent glass ceramics for optoelectronic applications. Incorporating $CaCl_2$ aids bulk crystal nucleation of chloroapatite at the expense of surface crystal nucleation. Figure 3 illustrates this by means of differential scanning calorimetry of fine <38 micron and frit 1-2mm glass particles. The temperature of the first crystallization peak $T_{c1}$ does not change with particle size indicating bulk nucleation and crystallization. Glasses W3-W6 all exhibited bulk crystal nucleation.

The starting oxychloride glass composition for the formation of apatite glass ceramics depends on the intended end application and the properties required. For example for the vitrification of radioactive waste a residual glass phase that is non degradable and chemically stable is required with as low a melting temperature as possible. In the case of apatite glass ceramics for up conversion processor eg. solar cells, fibre laser amplifiers etc again a relatively stable corrosion resistant residual glass phase is required. However for medical and dental applications a degradable residual glass phase is preferable if resorbtion is required.

The stability of the residual glass phase can be increased by starting with a glass with a slightly higher NC above 2.0 and by incorporating $Al_2O_3$ in the glass composition.

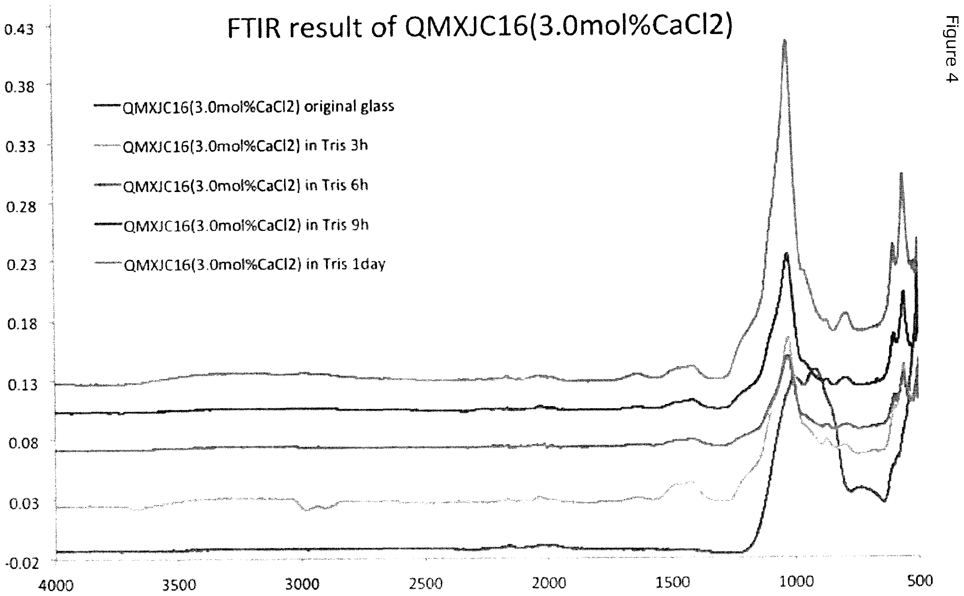

The dissolution behavior of the glasses was evaluated and their ability to form apatite. Glass QMXJC16 in powder form (75mg) with a particle size less than 38 microns was placed in a 100ml polyethene bottle in 50ml of Tris buffer at pH 7.4 for time periods up to 24 hours. The solution was filtered and the precipitate collected and the solution analysed by inductively coupled optical emission spectroscopy. The filtrate was characterized by X-ray diffraction, by ATR Fourier Transform Infra Red spectroscopy. Figure 4 shows the FTIR spectra of the filtrate as a function of immersion time. The formation of apatite is evidenced by the sharp bands at 1060, 600 and 560cm$^{-1}$. The degradation of the glass is clearly seen by the loss of non-bridging oxygen bands at about 900cm$^{-1}$ in the original glass.

All of the glasses formed apatite in Tris buffer and unlike the related fluorine-containing glasses there was no suppression of either the amount of apatite formed or the speed of its formation with increasing $CaCl_2$ content. Nor was there any formation of $CaCl_2$. In contrast high fluoride content glasses with more than 9 mole% $CaF_2$ tended to precipitate undesirable $CaF_2$ on dissolution. Apatite was also detected by XRD and $^{31}P$ solid state NMR. The apatite detected by XRD was not a chloroapatite, but a hydroxycarbonated apatite.

The choice of glass composition depends on the intended end application and the properties required. For example, for the vitrification of radioactive waste a residual glass phase that is non degradable and chemically stable is required with as low a melting temperature as possible. In the case of apatite glass ceramics for up conversion processors (e.g. solar cells, fibre laser amplifiers etc), again a relatively stable corrosion resistant residual glass phase is required. However, for medical and dental applications, a degradable residual glass phase is preferable if resorbtion is required.

Of the above examples, Compositions TP1 to TP2 are the most appropriate compositions for soft non-abrasive toothpastes. Compositions GCB1 to GCB3 are suitable for chloroapatite glasses and glass ceramic bone substitutes. Here the apatite phase that forms depends on the $CaF_2/CaCl_2$ content. Small amounts of MgO are added to improve the casting and viscous sintering characteristics of these glasses. Compositions QMRW1 to QMRW4 are suitable compositions for chloroapatite glass ceramics for radioactive waste vitrification. These compositions give a corrosion resistant residual glass phase following crystallization of the chloroapatite phase for the formation of apatite glass ceramics depends on the intended end application and the properties required. The stability of the residual glass phase can be increased by starting with a glass with a slightly higher NC above 2.0 and by incorporating $Al_2O_3$ in the glass composition. Compositions QMUP1 to QMUP4 are suitable base glasses for up conversion processors when suitably doped with rare earth elements at a concentration of 0.1 to 2.0 weight percent. Suitable rare earths may include: Pr, Pm, Sm, Eu, Gd,Tb, Dy, Ho ,Er, Tm ,Yb and Lu. Alternatively, elements including Mn and Sb.

The above embodiments have been described to illustrate the invention and are not intended to be limiting. The skilled person will be readily able to devise alternative embodiments without departing from the scope of the claims.

## Claims

1. A chlorine-containing silicate glass comprising $SiO_2$, at least 1.5 mole percent metal chloride and at least 10 mole percent of any one or more of MgO, SrO, BaO, and CaO, wherein the glass comprises less than 1 mole percent $Al_2O_3$ and the glass has a network connectivity (NC) in the range 2 to 3, wherein "network connectivity" is the average number of non-bridging oxygens per silicon, and "non-bridging oxygen" is an oxygen that is not shared by two $SiO_4$ tetrahedra.

2. A glass according to claim 1, wherein

i) the ratio of non-bridging oxygens to bridging oxygens is between 0.7 and 1.5, wherein "non-bridging oxygen" is an oxygen that is not shared by two $SiO_4$ tetrahedra and "bridging oxygen" is an oxygen that is shared by

two SiO$_4$ tetrahedra; and/or
ii) the glass comprises at least 2 mole percent metal chloride; and/or
iii) the glass comprises up to 50 mole percent metal chloride; and/or
iv) the glass comprises up to 60 mole percent of MgO, SrO, BaO, and CaO combined.

3. A glass according to any preceding claim, wherein the glass comprises either CaO, SrO or BaO and a source of phosphate, the glass preferably comprising at least 20 mole percent combined of CaO and SrO.

4. A glass according to any preceding claim, wherein the glass comprises a fluoride.

5. A glass according to any preceding claim, wherein

i) the glass comprises at least 20 mole percent SiO$_2$, and/or
ii) the glass comprises up to 60 mole percent SiO$_2$, preferably up to 50 mole percent SiOz, and/or
iii) the glass comprises at least 2 mole percent P$_2$O$_5$, preferably at least 4 mole percent P$_2$O$_5$, and/or
iv) the glass comprises up to 20 mole percent P$_2$O$_5$, preferably up to 12 mole percent P$_2$O$_5$, more preferably up to 10 mole percent P$_2$O$_5$ and most preferably up to 8 mole percent P$_2$O$_5$.

6. A glass according to claim 1 or claim 5, wherein the glass comprises up to 60 mole percent of MgO, SrO, BaO, and CaO,

7. A glass according to claim 6, wherein the glass comprises either CaO, SrO or BaO and a source of phosphate.

8. A glass according to any one of the preceding claims, wherein the glass comprises 2 to 12 mole percent MgO, MgCl$_2$ or MgF$_2$.

9. A glass according to claim 3, or claim 4 or claim 5 when dependent on claim 3, for use in dental applications or as a bone substitute.

10. A glass ceramic prepared by subjecting a glass according to any preceding claim to heat treatment.

11. A glass ceramic according to claim 10, wherein the glass is heated to a temperature between 300 and 900°C.

12. A glass ceramic according to claim 10 or claim 11, wherein the glass comprises either CaO, SrO or BaO and a source of phosphate, and the glass is heated until it crystallises to an apatite phase, the apatite preferably being chloroapatite or a mixed fluoro/chloroapatite.

13. A glass ceramic

i) prepared from a glass according to claim 3, or claim 4 or claim 5 when dependent on claim 3, for use in dental applications or as a bone substitute, or
ii) prepared from a glass according to claim 7 for use in radioactive waste vitrification.

14. A glass ceramic prepared from a glass according to claim 7 or claim 8 for use in up conversion applications or lamp phosphors, the glass ceramic being doped with one or more rare earth elements, wherein the rare earths are selected from Y, La, CePr, Pm, Sm, Eu, Gd,Tb, Dy, Ho ,Er, Tm ,Yb and Lu, or with transition elements including Mn that exhibit fluorescence.

15. A toothpaste comprising a glass according to claim 3, or claim 4 or claim 5 when dependent on claim 3, or a glass ceramic according to claim 12.

**Patentansprüche**

1. Chlorhaltiges Silikatglas, umfassend SiO$_2$, mindestens 1,5 Mol-% Metallchlorid und mindestens 10 Mol-% eines oder mehrerer der Substanzen MgO, SrO, BaO und CaO, worin das Glas weniger als 1 Mol-% Al$_2$O$_3$ umfasst und das Glas eine Netzwerkkonnektivität (NC) im Bereich von 2 bis 3 aufweist, worin "Netzwerkkonnektivität" die durchschnittliche Anzahl von nicht-verbrückenden Sauerstoffatomen pro Silicium ist und "nicht-verbrückender Sauerstoff" ein Sauerstoff ist, der nicht von zwei $SiO_4$-Tetraedern gemeinsam genutzt wird.

2. Glas nach Anspruch 1, worin

i) das Verhältnis von nicht verbrückenden Sauerstoffatomen zu verbrückenden Sauerstoffatomen zwischen 0,7 und 1,5 liegt, worin "nicht-verbrückender Sauerstoff" ein Sauerstoff ist, der nicht von zwei $SiO_4$-Tetraedern gemeinsam genutzt wird, und "verbrückender Sauerstoff' ein Sauerstoff ist, der von zwei $SiO_4$-Tetraedern gemeinsam genutzt wird, und/oder
ii) das Glas mindestens 2 Mol-% Metallchlorid enthält, und/oder
iii) das Glas bis zu 50 Mol-% Metallchlorid enthält, und/oder
iv) das Glas zusammen bis zu 60 Mol-% MgO, SrO, BaO und CaO enthält.

3. Glas nach einem der vorstehenden Ansprüche, worin das Glas entweder CaO, SrO oder BaO und eine Phosphatquelle enthält, worin das Glas vorzugsweise mindestens 20 Mol-% CaO und SrO zusammen enthält.

4. Glas nach einem der vorstehenden Ansprüche, worin das Glas ein Fluorid enthält.

5. Glas nach einem der vorstehenden Ansprüche, worin

i) das Glas mindestens 20 Mol-% $SiO_2$ enthält, und/oder
ii) das Glas bis zu 60 Mol-% $SiO_2$, vorzugsweise bis zu 50 Mol-% $SiO_2$, enthält, und/oder
iii) das Glas mindestens 2 Mol-% $P_2O_5$, vorzugsweise mindestens 4 Mol-% $P_2O_5$, enthält, und/oder
iv) das Glas bis zu 20 Mol-% $P_2O_5$, vorzugsweise bis zu 12 Mol-% $P_2O_5$, noch bevorzugter bis zu 10 Mol-% $P_2O_5$ und am meisten bevorzugt bis zu 8 Mol-% $P_2O_5$ enthält.

6. Glas nach Anspruch 1 oder Anspruch 5, worin das Glas bis zu 60 Mol-% MgO, SrO, BaO und CaO enthält.

7. Glas nach Anspruch 6, worin das Glas entweder CaO, SrO oder BaO und eine Phosphatquelle enthält.

8. Glas nach einem der vorstehenden Ansprüche, worin das Glas 2 bis 12 Mol-% MgO, $MgCl_2$ oder $MgF_2$ enthält.

9. Glas nach Anspruch 3 oder Anspruch 4 oder Anspruch 5, wenn diese von Anspruch 3 abhängen, zur Verwendung in zahnmedizinischen Anwendungen oder als Knochenersatz.

10. Glaskeramik, hergestellt durch Wärmebehandlung eines Glases nach einem der vorstehenden Ansprüche.

11. Glaskeramik nach Anspruch 10, worin das Glas auf eine Temperatur zwischen 300 und 900°C erhitzt wird.

12. Glaskeramik nach Anspruch 10 oder 11, worin das Glas entweder CaO, SrO oder BaO und eine Phosphatquelle enthält und das Glas erhitzt wird, bis es zu einer Apatitphase kristallisiert, vorzugsweise worin das Apatit Chlorapatit oder ein gemischtes Fluor/Chlorapatit ist.

13. Glaskeramik

i) hergestellt aus einem Glas nach Anspruch 3 oder Anspruch 4 oder Anspruch 5, wenn diese von Anspruch 3 abhängen, zur Verwendung in zahnmedizinischen Anwendungen oder als Knochenersatz, oder
ii) hergestellt aus einem Glas nach Anspruch 7 zur Verwendung bei der Verglasung radioaktiver Abfälle.

14. Glaskeramik, hergestellt aus einem Glas nach Anspruch 7 oder 8 zur Verwendung in Anwendungen zur Aufwärtsumwandlung oder Lampenphosphor, worin die Glaskeramik mit einem oder mehreren Seltenerdelementen dotiert ist, worin die Seltenerden ausgewählt sind aus Y, La, CePr, Pm, Sm, Eu, Gd, Tb, Dy, Ho, Er, Tm, Yb und Lu, oder mit Übergangselementen einschließlich Mn, die Fluoreszenz aufweisen.

15. Zahnpasta, welche ein Glas nach Anspruch 3, oder Anspruch 4 oder Anspruch 5, wenn diese von Anspruch 3 abhängen, oder eine Glaskeramik nach Anspruch 12 umfasst.

**Revendications**

1. Verre de silicate contenant du chlore, comprenant du $SiO_2$, au moins 1,5 pour cent molaire de chlorure métallique et au moins 10 pour cent molaire d'un ou de plusieurs parmi du MgO, du SrO, du BaO et du CaO, dans lequel le verre comprend moins de 1 pour cent molaire d'$Al_2O_3$ et le verre a une connectivité de réseau (NC) dans la plage 2 à 3, dans lequel la « connectivité de réseau » est le nombre moyen d'oxygènes non pontants par silicium, et « l'oxygène non pontant » est un oxygène qui n'est pas partagé par deux tétraèdres de $SiO_4$.

2. Verre selon la revendication 1, dans lequel

   i) le rapport entre oxygènes non pontants et oxygènes pontants est entre 0,7 et 1,5, dans lequel « l'oxygène non pontant » est un oxygène qui n'est pas partagé par deux tétraèdres de $SiO_4$ et « l'oxygène pontant » est un oxygène qui est partagé par deux tétraèdres de $SiO_4$ ; et/ou
   ii) le verre comprend au moins 2 pour cent molaire de chlorure métallique ; et/ou
   iii) le verre comprend jusqu'à 50 pour cent molaire de chlorure métallique ; et/ou
   iv) le verre comprend jusqu'à 60 pour cent molaire de MgO, SrO, BaO et CaO combinés.

3. Verre selon une quelconque revendication précédente, dans lequel le verre comprend soit du CaO, soit du SrO, soit du BaO et une source de phosphate, le verre comprenant préférentiellement au moins 20 pour cent molaire combinés de CaO et SrO.

4. Verre selon une quelconque revendication précédente, dans lequel le verre comprend un fluorure.

5. Verre selon une quelconque revendication précédente, dans lequel

   i) le verre comprend au moins 20 pour cent molaire de $SiO_2$, et/ou
   ii) le verre comprend jusqu'à 60 pour cent molaire de $SiO_2$, préférentiellement jusqu'à 50 pour cent molaire de $SiO_2$, et/ou
   iii) le verre comprend au moins 2 pour cent molaire de $P_2O_5$, préférentiellement au moins 4 pour cent molaire de $P_2O_5$, et/ou
   iv) le verre comprend jusqu'à 20 pour cent molaire de $P_2O_5$, préférentiellement jusqu'à 12 pour cent molaire de $P_2O_5$, plus préférentiellement jusqu'à 10 pour cent molaire de $P_2O_5$ et le plus préférentiellement jusqu'à 8 pour cent molaire de $P_2O_5$.

6. Verre selon la revendication 1 ou la revendication 5, dans lequel le verre comprend jusqu'à 60 pour cent molaire de MgO, SrO, BaO et CaO.

7. Verre selon la revendication 6, dans lequel le verre comprend soit du CaO, soit du SrO, soit du BaO et une source de phosphate.

8. Verre selon l'une quelconque des revendications précédentes, dans lequel le verre comprend[2] 2 à 12 pour cent molaire de MgO, de $MgCl_2$ ou de $MgF_2$.

9. Verre selon la revendication 3, ou la revendication 4 ou la revendication 5 lorsqu'elle dépend de la revendication 3, pour utilisation dans des applications dentaires ou comme substitut osseux.

10. Vitrocéramique préparée en soumettant un verre selon une quelconque revendication précédente à un traitement thermique.

11. Vitrocéramique selon la revendication 10, dans laquelle le verre est chauffé à une température entre 300 et 900 °C.

12. Vitrocéramique selon la revendication 10 ou la revendication 11, dans laquelle le verre comprend soit du CaO, soit du SrO, soit du BaO et une source de phosphate, et le verre est chauffé jusqu'à ce qu'il cristallise en une phase apatite, l'apatite étant préférentiellement une chloroapatite ou un mélange fluoro/chloroapatite.

13. Vitrocéramique

   i) préparée à partir d'un verre selon la revendication 3, ou la revendication 4 ou la revendication 5 lorsqu'elle dépend de la revendication 3, pour utilisation dans des applications dentaires ou comme substitut osseux, ou ii) préparée à partir d'un verre selon la revendication 7, pour utilisation dans la vitrification des déchets radioactifs.

14. Vitrocéramique préparée à partir d'un verre selon la revendication 7 ou la revendication 8 pour utilisation dans des applications de conversion vers le haut ou dans des phosphores pour lampe, la vitrocéramique étant dopée avec un ou plusieurs éléments des terres rares, dans laquelle les terres rares sont choisies parmi Y, La, CePr, Pm, Sm, Eu, Gd, Tb, Dy, Ho, Er, Tm, Yb et Lu, ou avec des éléments de transition incluant du Mn qui présentent une fluorescence.

15. Dentifrice comprenant un verre selon la revendication 3, ou la revendication 4 ou la revendication 5 lorsqu'elle dépend de la revendication 3, ou une vitrocéramique selon la revendication 12.

DSC Results for Glass Frits of CaX$_2$ Series Glasses

Y=-7.6834X+785.17

R$^2$=0.98123

Crystallised to FAP

Y=-7.1476X+784.6

R$^2$=0.99666

◆ CaCl$_2$ series

■ CaF$_2$ series

▲ CaF$_2$, CaCl$_2$ series

✕ P free CaCl$_2$ series

Glass Transition Temperature (T$_g$ °C)

CaX$_2$ content (mol%)

EP 2 978 383 B1

Figure 2

Figure 3

Figure 4

FTIR result of QMXJC16(3.0mol%CaCl2)

— QMXJC16(3.0mol%CaCl2) original glass
— QMXJC16(3.0mol%CaCl2) in Tris 3h
— QMXJC16(3.0mol%CaCl2) in Tris 6h
— QMXJC16(3.0mol%CaCl2) in Tris 9h
— QMXJC16(3.0mol%CaCl2) in Tris 1day

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9625203 A1


**Non-patent literature cited in the description**

- **VANCE et al.** *J. Nuclear Materials,* 2012, vol. 420, 396-404